(19) **Europäisches Patentamt**
**European Patent Office**
**Office européen des brevets**

(11) **EP 2 198 703 A2**

(12) **EUROPEAN PATENT APPLICATION**

(43) Date of publication:
**23.06.2010 Bulletin 2010/25**

(51) Int Cl.:
*A01K 43/08* (2006.01)  *B07C 5/16* (2006.01)
*G01G 9/00* (2006.01)  *G01N 23/04* (2006.01)
*G01N 33/02* (2006.01)  *G01N 33/08* (2006.01)
*G01N 33/12* (2006.01)  *G01G 11/00* (2006.01)
*B07C 5/18* (2006.01)

(21) Application number: **09179347.1**

(22) Date of filing: **15.12.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **16.12.2008 JP 2008320096**

(71) Applicant: **ISHIDA CO., Ltd.**
**Kyoto-shi,**
**Kyoto 606-8392 (JP)**

(72) Inventors:
• **Tsuno, Masao**
  **Shiga 520-3026 (JP)**
• **Miyazaki, Kiyoshi**
  **Shiga 520-3026 (JP)**
• **Yamamoto, Shinya**
  **Shiga 520-3026 (JP)**
• **Yamamoto, Yasuji**
  **Shiga 520-3026 (JP)**
• **Yamamoto, Yasuhiro**
  **Shiga 520-3026 (JP)**
• **Matsumura, Tetsuji**
  **Shiga 520-3026 (JP)**
• **Katayama, Koji**
  **Okayama 701-1152 (JP)**

(74) Representative: **Kastel, Stefan et al**
**Flügel Preissner Kastel Schober**
**Nymphenburger Strasse 20a**
**80335 München (DE)**

(54) **Apparatus for determining the mass/weight of articles on a conveyer belt by X-ray imaging and for subsequent sorting of the articles by mass/weight**

(57)     An X-ray inspection (10) apparatus includes an X-ray radiating part, an X-ray detecting part, a mass estimation unit and a mass class determination unit. The X-ray radiating part is configured and arranged to radiate X-rays to an inspection target (8). The X-ray detecting part is configured and arranged to detect the X-rays radiated from the X-ray radiating part that transmitted through the inspection target (8). The mass estimation unit is configured to estimate a mass of the inspection target based on an amount of the X-rays detected by the X-ray detecting part. The mass class determination unit is configured to determine which mass class among a plurality of mass classes (53a,53b,...53g,54) within a preset range the inspection target (8) belongs to based on the mass of the inspection target estimated by the mass estimation unit.

F I G. 1

## Description

CROSS-REFERENCE TO RELATED APPLICATIONS

This application claims priority to Japanese Patent Application No. 2008-320096, filed on December 16, 2008. The entire disclosure of Japanese Patent Application No. 2008-320096 is hereby incorporated herein by reference.

BACKGROUND

Field of the Invention

The present invention relates to an X-ray inspection apparatus that is capable of estimating the mass of an inspection target by radiating X-rays to the inspection target and detecting the X-rays that transmit there through, and then classifying the inspection target based on its mass.

Background Information

Known in the conventional art is an X-ray inspection apparatus that measures a physical quantity of a target of the inspection and determines whether that inspection target is good or bad. For example, Japanese Patent Application Publication No. 2006-308467 discloses one that is provided with an X-ray source, an X-ray detecting part, a data processing unit, a display unit, an area extraction processing unit, a mass calculation unit, and a display data generation unit. The area extraction processing unit extracts some of the detection information from the X-ray detecting part and thereby causes an X-ray image generating unit of the data processing unit to generate the X-ray image of a physical quantity measurement area at which the physical quantity is to be measured, at least a portion that corresponds to the background of the area being excluded on the X-ray image. Based on the detection information extracted by the area extraction processing unit, the mass calculation unit derives the physical quantity that corresponds to the size or the mass of a workpiece W. The display data generation unit associates and displays on the display unit the X-ray image of the physical quantity measurement area and a graph display element that indicates the physical quantity computed by the mass calculation unit.

SUMMARY

In recent years, it has become desirable to use the measured physical quantity of an inspection target for purposes other than determining whether the inspection target is good or bad.

Taking the above problems into consideration, it is an object of the present invention to provide an X-ray inspection apparatus that is capable of being used to classify an inspection target based on the grade or class of its estimated mass.

An X-ray inspection apparatus according to one aspect of the present invention includes an X-ray radiating part, an X-ray detecting part, a mass estimation unit and a mass class determination unit. The X-ray radiating part is configured and arranged to radiate X-rays to an inspection target. The X-ray detecting part is configured and arranged to detect the X-rays radiated from the X-ray radiating part that transmitted through the inspection target. The mass estimation unit is configured to estimate a mass of the inspection target based on an amount of the X-rays detected by the X-ray detecting part. The mass class determination unit is configured to determine which mass class among a plurality of mass classes within a preset range the inspection target belongs to based on the mass of the inspection target estimated by the mass estimation unit.

BRIEF DESCRIPTION OF THE DRAWINGS

Referring now to the attached drawings which form a part of this original disclosure:

FIG. 1 is a simplified top plan view of an X-ray inspection apparatus according to an embodiment of the present invention.

FIG. 2 is a simplified front view schematically showing an overall configuration of an X-ray inspection unit in the X-ray inspection apparatus shown in FIG. 1.

FIG. 3 is a perspective view showing the internal configuration of a shield box in the X-ray inspection unit shown in FIG. 2.

FIG. 4 is an exploded perspective view for explaining the configuration of a conveyor shown in FIG. 3.

FIG. 5 is a simplified cross sectional view showing the configuration of a rejecter part in a sorting unit of the X-ray inspection apparatus shown in FIG. 1.

FIG. 6 is a block diagram of the functional configuration of the X-ray inspection unit shown in FIG. 1.

FIG. 7 is a block diagram showing the functional configuration that provides a computer shown in FIG. 2 with the inspection function.

FIG. 8 is a block diagram that shows the functional configuration of a mass inspection unit and a shape inspection unit shown in FIG. 7.

DETAILED DESCRIPTION OF EMBODIMENTS

Selected embodiments will now be explained with reference to the drawings. It will be apparent to those skilled in the art from this disclosure that the following descriptions of the embodiments are provided for illustration only and not for the purpose of limiting the invention as defined by the appended claims and their equivalents.

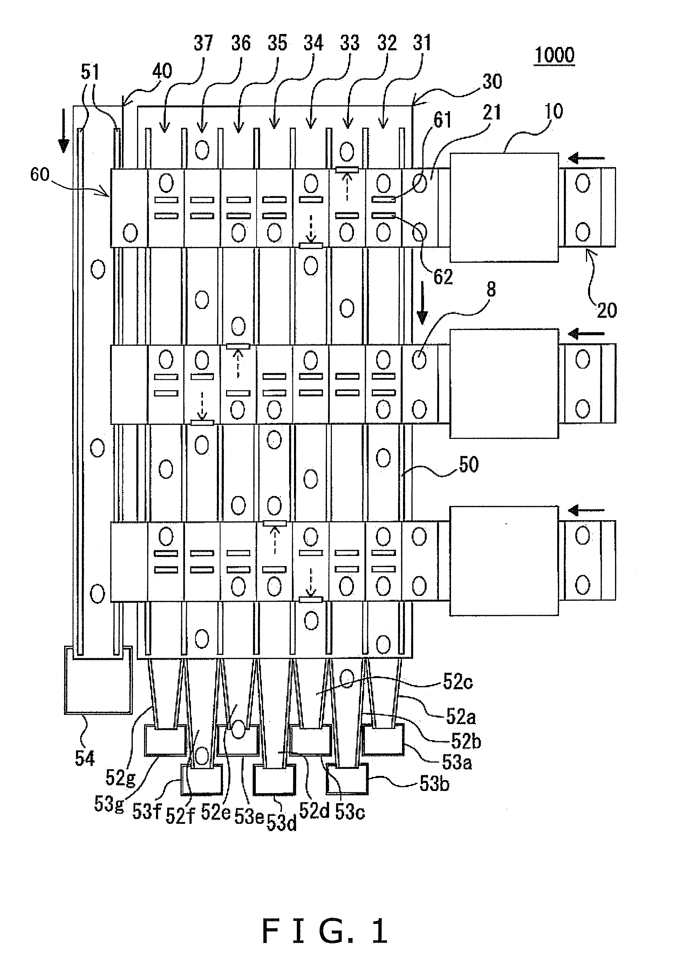

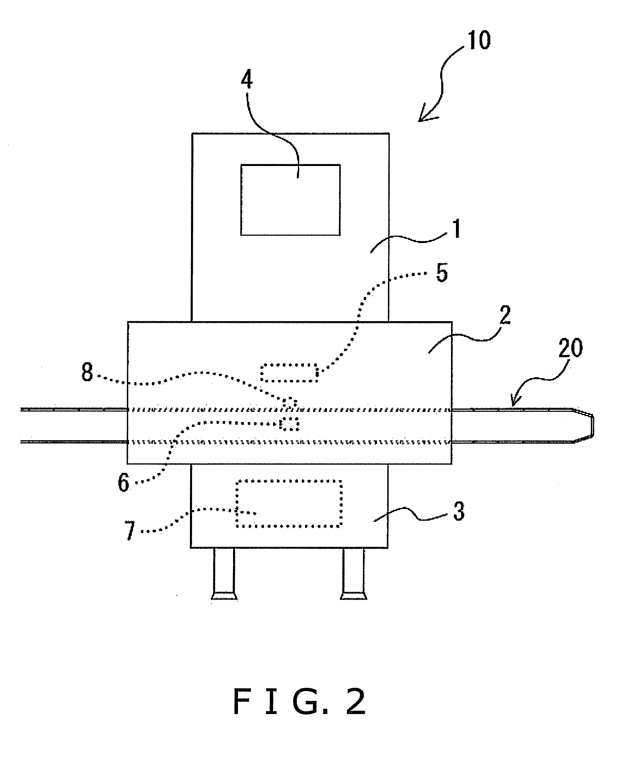

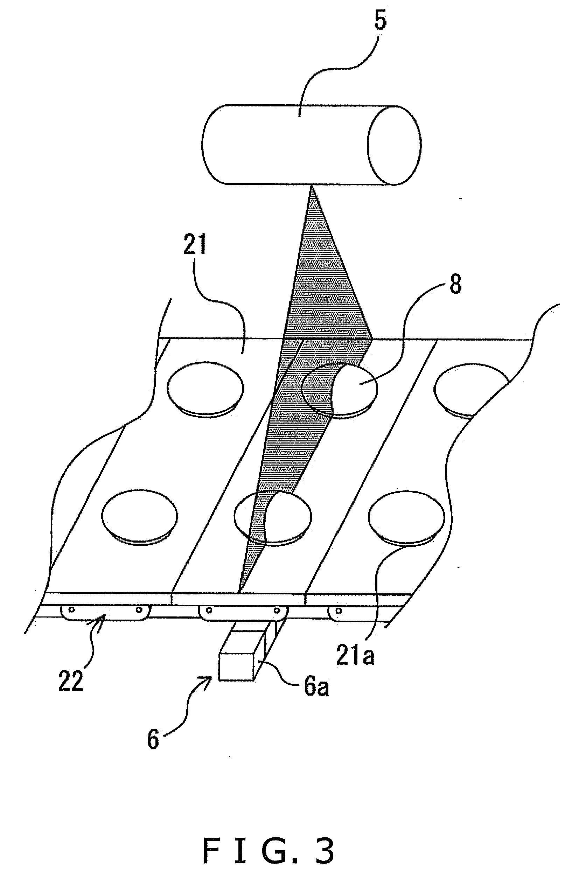

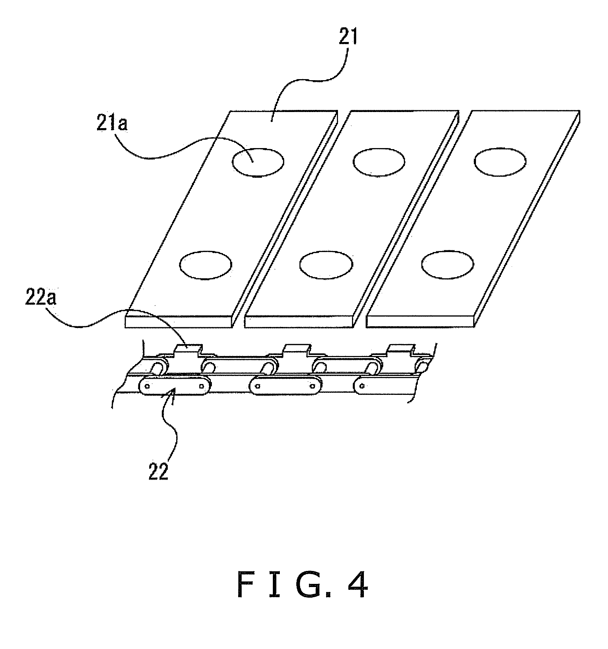

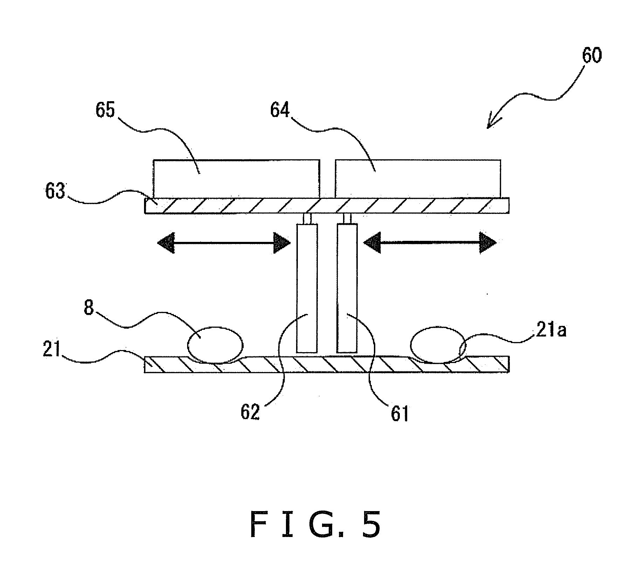

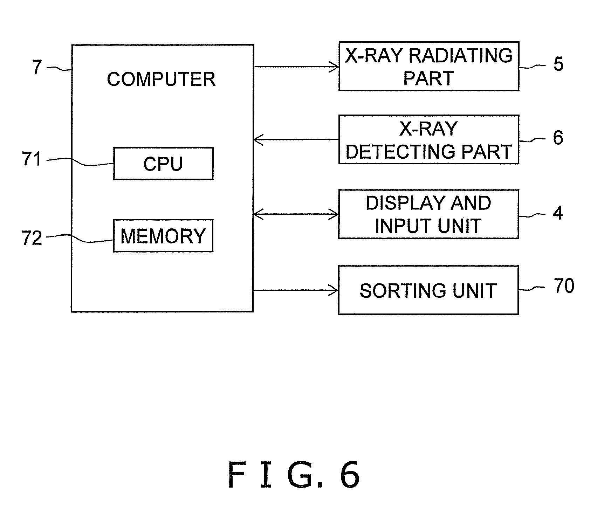

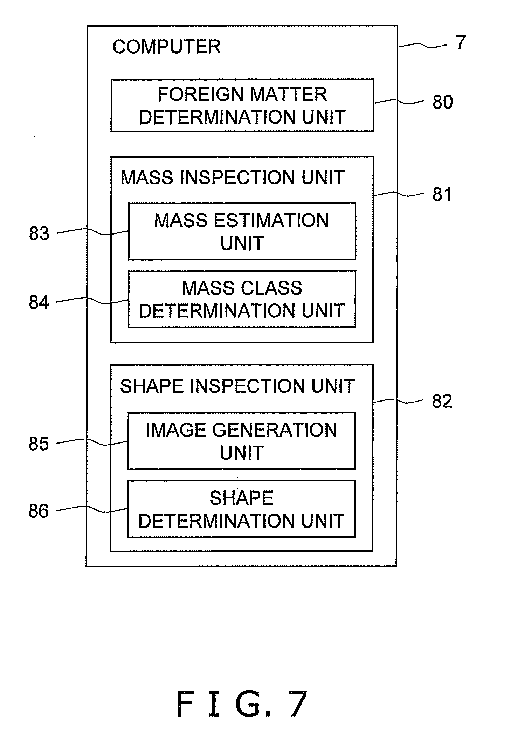

The following text explains an X-ray inspection apparatus according to an embodiment of the present invention, referencing the drawings. FIG. 1 is a schematic configuration diagram of the X-ray inspection apparatus according to the embodiment of the present invention. In FIG. 1, the solid line arrows indicate the directions of flow of conveyors and articles, and the dotted line arrows schematically indicate the operation states of plate members that constitute parts of rejecter parts. FIG. 2 is a front view that schematically shows the entire configuration of an X-ray inspection unit in the X-ray inspection apparatus of FIG. 1. FIG. 3 is an oblique view that shows the internal configuration of a shield box in the X-ray inspection unit of FIG. 2. FIG. 4 is an oblique exploded view for explaining the configuration of the conveyor shown in FIG. 3. FIG. 5 is a cross sectional view that shows the configuration of a rejecter part in a sorting unit of the X-ray inspection apparatus in FIG. 1. FIG. 6 is a block diagram that shows the functional configuration of an X-ray inspection unit 10. FIG. 7 is a block diagram that shows the inspection function performed by a computer 7.

An X-ray inspection apparatus 1000 shown in FIG. 1 includes X-ray inspection units 10, conveyors 20, 30, 40, guide members 50, 51, feeders 52a, 52b, 52c, 52d, 52e, 52f, 52g, boxes 53a, 53b, 53c, 53d, 53e, 53f, 53g, a defective product collection box 54, and rejecter parts 60 (only parts of which are shown in FIG. 1). In addition, along the transport direction of the conveyor 30, the X-ray inspection apparatus 1000 is further provided with two additional rows of the X-ray inspection unit 10 and the conveyor 20, which are configured the same as the first row.

As shown in FIG. 2, each of the X-ray inspection units 10 includes an upper part casing 1, a shield box 2, and a lower part casing 3. The upper part casing 1 is provided with a monitor that includes a touch panel function, namely, it is provided with a display and input unit 4. In addition, an X-ray radiating part 5 and an X-ray detecting part 6 are provided inside the shield box 2, and the conveyor 20 is provided and disposed such that it passes through the interior of the shield box 2. The X-ray radiating part 5 radiates X-rays to inspection targets 8 on the conveyor 20. The X-ray detecting part 6 is provided inside the conveyor 20 and detects the X-rays that were radiated from the X-ray radiating part 5 and transmitted through the inspection targets 8. The shield box 2 has a function that prevents the X-rays from leaking to the exterior thereof. To control the operation of the X-ray inspection unit 10 and to perform data processing, the computer 7 is provided and disposed inside the lower part casing 3. Furthermore, examples of the inspection targets 8 are foodstuffs (e.g., fruits and vegetables, shellfishes, peeled boiled eggs, and mushrooms), but the present invention is not limited thereto.

The conveyor 20 is provided and disposed such that it extends above the conveyor 30 from the corresponding X-ray inspection unit 10 through the interior of the shield box 2 and on to an end part of the conveyor 40. In addition, as shown in FIG. 3 and FIG. 4, the conveyor 20 includes plates 21 each of which has recessed parts 21a on its front surface and a chain belt 22 that has support parts 22a, which fix and support the plates 21 from their rear surface sides. Furthermore, in FIG. 4, the chain belt 22 is shown only on the near side, but actually the far side is configured symmetrically to the near side around the center part of the plates 21. In addition, the chain belts 22 are provided extending across and engaged with two chain sprockets (not shown). Each of the chain sprockets is connected to a motor (not shown), the rotation of which drives the chain belts 22.

The conveyor 30 transports the inspection targets 8 in a direction substantially perpendicular to the transport direction of the conveyors 20 toward the feeders 52a, 52c, 52e, 52g (i.e., the short types) and the feeders 52b, 52d, 52f (i.e., the long types). A plurality of the guide members 50 are provided in the transport direction such that they are suspended above the conveyor 30; furthermore, the guide members 50 form lanes 31-37, which guide the classified inspection targets 8 to the feeders 52a-52g.

The conveyor 40 is provided parallel to the conveyor 30 and transports the inspection targets 8 in the same direction as the transport direction of the conveyor 30. The two guide members 51 are provided in the transport direction such that they are suspended above the conveyor 40, and thereby the inspection targets 8 determined to be defective articles can be guided to the defective product collection box 54.

Each of the rejecter parts 60 includes: plate members 61, 62; and drive parts 64, 65, which are provided to a top plate 63 and, based on commands from the computer 7, are respectively capable of freely moving the plate members 61, 62 reciprocatively in the directions of the arrows shown in FIG. 5. Furthermore, the plate members 61, 62 are provided such that they are respectively suspended from the drive parts 64, 65 via slits (not shown) provided to the top plate 63.

Furthermore, the conveyors 20, 30, 40, the guide members 50, 51, and the rejecter parts 60 constitute part of a sorting unit 70 (FIG. 6).

As shown in FIG. 6, the computer 7 includes a CPU 71 and memory 72, such as ROM, RAM and the like, that is capable of being referenced by the CPU 71. The X-ray radiating part 5, the X-ray detecting part 6, the display and input unit 4, and the sorting unit 70 are connected to the computer 7. In addition, as shown in FIG. 7, the computer 7 includes a foreign matter determination unit 80, a mass inspection unit 81, and a shape inspection unit 82. The foreign matter determination unit 80 determines whether foreign matter has contaminated any of the inspection targets 8. The mass inspection unit 81 inspects whether the mass of each of the inspection targets 8 is within a target range (i.e., within a permissible range) and the shape inspection unit 82 inspects, for example, whether a crack or a chip is present in any of the inspection targets 8. Furthermore, each of the inspection functions shown in FIG. 7 is implemented by inspection programs, which are stored in the memory 72 shown in FIG. 6, executed by the CPU 71. The following text discusses the foreign matter determination unit 80, the mass inspection unit 81, and the shape inspection unit 82 in detail.

First, the foreign matter determination unit 80 will be explained. Referencing FIG. 3, the X-ray detecting part 6, which includes a plurality of X-ray detecting elements 6a, detects X-rays radiated from the X-ray radiating part 5 that transmit through the inspection targets 8 (i.e., transmitted X-rays). Here, if foreign matter has not contaminated any of the inspection targets 8, then the intensity distribution of the transmitted X-rays detected by the plurality of the X-ray detecting elements 6a will be substantially constant. However, if foreign matter has contaminated any of the inspection targets 8, then the amount of the transmitted X-rays at the location of the foreign matter contamination will decrease, which will consequently generate a negative peak in the intensity distribution of the transmitted X-rays that corresponds to the location of the foreign matter contamination. Accordingly, based on X-ray detection data sent from the X-ray detecting part 6, the foreign matter determination unit 80 determines whether a peak has been generated in the intensity distribution of the transmitted X-rays, and thereby determines whether foreign matter contamination is present in the corresponding inspection target 8. When one of the inspection targets 8 is determined to be contaminated with foreign matter, it does not undergo inspections by the mass inspection unit 81 and the shape inspection unit 82 (discussed below) and is instead sorted as a defective article by the sorting unit 70 shown in FIG. 6.

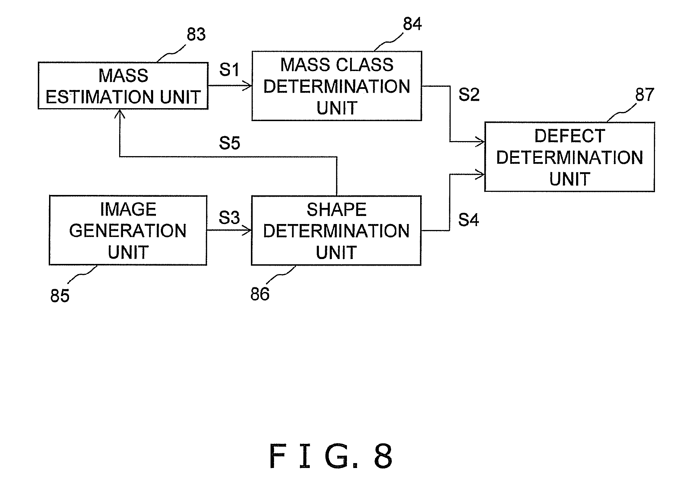

FIG. 8 is a block diagram that shows the functional configuration of the mass inspection unit 81 and the shape inspection unit 82 shown in FIG. 7. The mass inspection unit 81 includes a mass estimation unit 83 and a mass class determination unit 84, and the shape inspection unit 82 includes an image generation unit 85 and a shape determination unit 86. In addition, the mass class determination unit 84 and the shape determination unit 86 are connected to a defect determination unit 87, which is implemented in the computer 7.

The mass estimation unit 83 estimates the mass of the relevant inspection target 8 based on the amount of X-rays that transmit through this inspection target 8 and are detected by the X-ray detecting part 6 shown in FIG. 2 and outputs data S1 related to an estimated mass M. The following text explains this in detail. Referencing FIG. 3, the X-ray detecting part 6 detects the amount of X-rays radiated from the X-ray radiating part 5 that transmit through the relevant inspection target 8. Specifically, the X-ray detecting elements 6a that constitute the X-ray detecting part 6 detect a brightness $I$ of the transmitted X-rays. Here, each of the X-ray detecting elements 6a detects the brightness $I$ with a detection gray scale of 256 gradations, wherein, for example, the maximum brightness, namely, white, is assigned "255" and the minimum brightness, namely, black, is assigned "0." A detection value related to the brightness $I$ from each of the X-ray detecting elements 6a is inputted to the mass estimation unit 83 shown in FIG. 8, and the mass estimation unit 83 computes an estimated mass $m$ based on the brightness $I$ from each of the X-ray detecting elements 6a using equation (1) below to estimate the mass.

$$m = ct = -c/\mu \times \ln(I/I0) = -\alpha ln(I/I0) \qquad (1)$$

Here, $c$ is a coefficient for converting the thickness of the substance to mass, $t$ is the thickness of the substance, $I0$ is the brightness of the X-rays when there is no substance, $I$ is the brightness of the X-rays after transmitting through substance, and $\mu$ is a linear absorption coefficient. In addition, $\alpha$ is a parameter that is assigned the value of $c/\mu$; furthermore, an appropriate value for each type of the inspection targets 8 is derived in advance by performing pre-inspections using a plurality of samples with known masses, and those values are stored in the memory 72 shown in FIG. 6.

For each of the pixels (i.e., for each of the X-ray detecting elements 6a), the mass estimation unit 83 uses the abovementioned equation (1) to convert the brightness $I$ to the estimated mass $m$. Furthermore, while the conveyor 20 transports the inspection targets 8, the detection of the brightness $I$ by each of the X-ray detecting elements 6a and the conversion of the brightness $I$ to the estimated masses $m$ are performed repetitively. Thereby, the estimated mass $m$ is derived for every pixel of the relevant inspection target 8, and the estimated mass $M$ of the entire relevant inspection target 8 is derived by summing all of these estimated masses $m$.

Based on the data S1 input from the mass estimation unit 83, the mass class determination unit 84 determines (1) which mass class of prescribed mass classes (for example, six levels which a prescribed range is divided into and which is set in advance for each type of the inspection targets 8, each level falling within the prescribed range) the mass of the inspection target 8 belongs to or (2) if the mass of the relevant inspection target 8 does not belong to any of the mass classes within the prescribed range, that the inspection target 8 is abnormal and outputs data S2 related to that determination result. The following text explains this more specifically. An upper limit value and a lower limit value of a target range (i.e., a permissible range) for each mass class is preset and stored in the memory 72 shown in FIG. 6, for each type of the inspection targets 8. The mass class determination unit 84 compares the estimated mass $M$ expressed by the data S1 with the upper limit value and the lower limit value of each of the mass classes mentioned above; if the estimated mass $M$ is less than the upper limit value and greater than or equal to the lower limit value of any of the mass classes, then the mass class determination unit 84 determines that the mass of that inspection target 8 belongs to that mass class. However, if the estimated mass $M$ does not belong to any of the mass classes, then the mass of that inspection target 8 is determined to be abnormal.

Based on the amount of transmitted X-rays detected by the X-ray detecting part 6 shown in FIG. 2, the image generation unit 85 generates an X-ray transmission image and outputs image data S3 related to that X-ray transmission image. Specifically, as mentioned above, each of the X-ray detecting elements 6a that constitute the X-ray detecting part 6 detects the brightness $I$ with a detection gray scale of 256 gradations; for example, the maximum brightness, namely, white, is assigned "255" and the minimum brightness, namely, black, is assigned "0." Thereby, one line data included in image data for the inspection target 8 is generated. Furthermore, while the conveyor 20 is transporting the inspection target 8, the detection of the brightness I by each of the X-ray detecting elements 6a is performed repetitively for each line. Thereby, the brightness $I$ for every pixel of the inspection target 8 is derived and the X-ray transmission image of the entire inspection target 8 (i.e., the image data S3) is generated.

Based on the image data S3 input from the image generation unit 85, the shape determination unit 86 determines whether the shape of the inspection target 8 is normal or abnormal and outputs data S4 related to that determination result. Consider an example wherein data related to the upper limit value and the lower limit value of the perimetric length of a nondefective article is prestored in the memory 72 shown in FIG. 6. In that case, the shape determination unit 86 extracts the edge from the X-ray transmission image of the inspection target 8 expressed by the image data S3 and compares the total length of the extracted edge with the abovementioned upper and lower limit values. If the total length of the edge is less than or equal to the upper limit value and greater than or equal to the lower limit value, then the shape determination unit 86 determines that the shape of that inspection target 8 is normal; however, if the total length of the edge is greater than the upper limit value or less than the lower limit value, then the shape determination unit 86 determines that the shape of that inspection target 8 is abnormal. For example, if there is a crack in the inspection target 8, then the total length of the edge will increase; consequently, the total length of the edge will be greater than the abovementioned upper limit value, which makes it possible to identify the inspection targets 8 that have cracks.

Consider another example wherein data related to the upper and lower limit values of the upper surface area of a nondefective article is prestored in the memory 72 shown in FIG. 6. In that case, the shape determination unit 86 derives the upper surface area of the relevant inspection target 8 from the X-ray transmission image expressed by the image data S3 and compares that derived upper surface area value with the abovementioned upper limit value and lower limit value. If the value of the upper surface area is less than or equal to the upper limit value and greater than or equal to the lower limit value, then the shape determination unit 86 determines that the shape of that inspection target 8 is normal; however, if the value of the upper surface area is greater than the upper limit value or less than the lower limit value, then the shape determination unit 86 determines that the shape of that inspection target 8 is abnormal. For example, if the inspection target 8 has a chip, then the upper surface area will decrease, and consequently the value of the upper surface area will become less than the abovementioned lower limit value, which makes it possible to identify the inspection targets 8 that have chips.

Consider yet another example wherein image data of a template image related to the contour shape of a nondefective article is prestored in the memory 72 shown in FIG. 6. In that case, the shape determination unit 86 performs a template matching process, which uses the abovementioned template image, within the X-ray transmission image of the inspection target 8 expressed by the image data S3. If the degree of similarity between the inspection target 8 and the template image is greater than or equal to a prescribed level, then the shape determination unit 86 determines that the shape of that inspection target 8 is normal; however, if the degree of similarity is less than the prescribed level, then the shape determination unit 86 determines that the shape of that inspection target 8 is abnormal. For example, if the relevant inspection target 8 has a crack or a chip, then the difference from the contour shape of the nondefective article will increase, and consequently the degree of similarity will become less than the abovementioned prescribed level, which makes it possible to identify the inspection targets 8 that have cracks or chips.

Consider yet another example wherein image data of a template image related to the size of a nondefective article that has a prescribed shape is prestored in the memory 72 shown in FIG. 6. In that case, the shape determination unit 86 performs the template matching process, which uses the abovementioned template image, within the X-ray transmission image of the inspection target 8 expressed by the image data S3. If the degree of similarity between the inspection target 8 and the template image is greater than or equal to the prescribed level, then the shape determination unit 86 determines that the size of that inspection target 8 is normal; however, if the degree of similarity is less than the prescribed level, then the shape determination unit 86 determines that the size of that inspection target 8 is abnormal. For example, if the inspection target 8 is too large or too small, then the difference from the size of the nondefective article of a prescribed shape will increase, and consequently the degree of similarity will become less than the prescribed level, which makes it possible to identify the inspection targets 8 that are significantly different in size from the nondefective article.

Consider yet another example wherein image data of a template image related to the gray-scale pattern (e.g., of gray-scale peaks and gray-scale deviations) of a nondefective article is prestored in the memory 72 shown in FIG. 6. In that case, the shape determination unit 86 performs the template matching process, which uses the abovementioned template image, within the X-ray transmission image of the inspection target 8 expressed by the image data S3. Specifically, first, the degree of similarity is determined by comparing the abovementioned template image, which is an image of the entire area or a prescribed area narrower than the entire area, with the X-ray transmission image of each of the inspection targets 8. Furthermore, the shape determination unit 86 counts how many pixels are similar to or match the reference image within the aforementioned area. If the degree of similarity between the relevant inspection target 8 and the template image is greater than or equal to a prescribed level, then the shape determination unit 86 determines that the shape of that inspection target 8 is normal; however, if the degree of similarity is less than the prescribed level, then the shape determination unit 86 determines that the shape of that inspection target 8 is abnormal. For example, if the inspection target 8 has a crack or a chip, then the difference from the gray-scale pattern of the nondefective article will increase, and consequently the degree of similarity will become less than the abovementioned prescribed level, which makes it possible to identify the inspection targets 8 that have cracks or chips.

In the defect determination unit 87, the data S2 (i.e., the mass class or the abnormal state that was determined) is input from the mass class determination unit 84, and the data S4 (i.e., the normal/abnormal state of the shape) is input from the shape determination unit 86. Furthermore, based on the data S2, S4, if at least one of the determination results produced by the mass class determination unit 84 and the shape determination unit 86 is abnormal, then the relevant inspection target 8 is determined to be a defective article. Specifically, if the determination result produced by the mass class determination unit 84 is abnormal, then none of the rejecter parts 60 shown in FIG. 1 and FIG. 5 pushes the relevant inspection target 8 out onto the conveyor 30, the relevant inspection target 8 is conveyed to and dropped onto the conveyor 40, and thereby that inspection target 8 is eliminated from the conveyor 20. In particular, the front surface of each of the plates 21 is labeled with an individual number, and the computer 7 uses an image recognizing means, such as a CCD camera, to recognize those individual numbers and thereby can continuously ascertain the position of each of the plates 21. Furthermore, when the plate 21 (whereon the inspection target 8 that was determined to have any of prescribed mass classes is mounted) is being transported by the conveyor 20 over the lane 31 shown in FIG. 1, the relevant rejecter part 60 is driven in accordance with a command from the computer 7, and thereby the inspection target 8 is eliminated from the conveyor 20 and sorted. If the determination result produced by the mass class determination unit 84 corresponds to any of the mass classes, then, as described above, the computer 7 uses the individual number and the positional information of the relevant plate 21 to make the relevant rejecter part 60 push out the relevant inspection target 8 into the lane on the conveyor 30 that corresponds to that mass class (i.e., any one of the lanes 32-37 in FIG. 1), and thereby the inspection targets 8 are sorted. In addition, if the determination result produced by the shape determination unit 86 is abnormal, then, as described above, the computer 7 does not make any of the relevant rejecter parts 60 push out the relevant inspection target 8 onto the conveyor 30, rather, makes the relevant inspection target 8 drop onto the conveyor 40, thereby eliminating it from the conveyor 20. Each of the inspection targets 8 thus sorted is transported to one of the boxes 53a, 53b, 53c, 53d, 53e, 53f, 53g or to the defective product collection box 54. Furthermore, the determination results produced by the defect determination unit 87 are displayed on the display and input unit 4 shown in FIG. 2.

The present embodiment exhibits the following effects. Namely, the X-ray inspection apparatus 1000 provided by the present embodiment is capable of classifying the given inspection target 8 into one of the mass classes based on the estimated mass of the inspection target 8.

In addition, the X-ray inspection apparatus 1000 provided by the present embodiment is capable of sorting the abnormal inspection target 8 that does not belong to any of the mass classes because the mass class determination unit 84 can determine that the given inspection target 8 is abnormal. Therefore, the X-ray inspection apparatus 1000 can be provided that can be used to not only to determine whether the inspection target 8 is good or bad, but also to classify the inspection target 8 according to its estimated mass.

Furthermore, the X-ray inspection apparatus 1000 provided by the present embodiment is capable of sorting the inspection targets 8 into nondefective articles and defective articles based on their shapes because the image generation unit 85 and the shape determination unit 86 are provided. Thus, it is possible to provide the X-ray inspection apparatus 1000 that can not only be used to classify the inspection target 8 as a nondefective article or a defective article based on its shape, but also to classify the inspection target 8 into one of the mass classes based on its mass.

In addition, because the foreign matter determination unit 80 is provided, the X-ray inspection apparatus 1000 provided by the present embodiment is capable of not only easily selecting the inspection target 8 that contains uneatable foreign matter or the like, but also classifying the inspection target 8 into one of the mass classes according to its mass.

In addition, because the sorting unit 70 is provided, if the mass class determination unit 84 determines that the given inspection target 8 belongs to any one of the mass classes, then that inspection target 8 can be sorted into the corresponding mass class (specifically, into the corresponding lane of the lanes 32-37); furthermore, if the mass class determination unit 84 determines that the given inspection target 8 is abnormal, then that inspection target 8 can be sorted onto the conveyor 40 as a defective article. In addition, if the shape determination unit 86 determines that the shape of the given inspection target 8 is abnormal, then the sorting unit 70 can sort that inspection target 8 onto the conveyor 40 as a defective article; furthermore, if the foreign matter determination unit 80 determines that the given inspection target 8 contains foreign matter, then that inspection target 8 can be sorted into the lane 31 as a defective article.

Furthermore, it is understood that changes to the design may be effected without departing from the spirit and scope of the claims and that the present invention is not limited to the embodiments and modified examples explained above. For example, the X-ray radiating part 5 in the above embodiments radiates X-rays in one direction alone; however, a modification may be effected such that the X-ray radiating part 5 radiates X-rays to each of the inspection targets 8 from the same direction toward two or more locations of the inspection target 8, or from two or more directions toward at least one location of the inspection target 8, and the mass estimation unit 83 additionally estimates the mass of the useful portion of each of the inspection targets 8 based on the amount of X-rays detected by the X-ray detecting part 6; furthermore, based on the mass of the useful portion of the given inspection target 8 estimated by the modified mass estimation unit 83, the mass class determination unit 84 may determine which class, among the mass classes within a prescribed range set beforehand for each type of the inspection targets 8, the given inspection target 8 belongs to. Thereby, it is possible to classify the valuable useful portion of the given inspection target 8 according to its mass class, even if the contents of the inspection target 8 cannot be seen from outside. Here, examples of the useful portion of the given inspection target 8 include the edible portion of a fruit, vegetable, or shellfish, the egg yolk of an egg with shell, the egg yolk of a peeled hardboiled egg, a pearl that has grown inside a pearl oyster, and the edible portion of a frozen crab claw; however, the present invention is not limited thereto.

In addition, if the given inspection target 8 is, for example, a fruit or vegetable, then the shape determination unit 86 may determine, based on the X-ray transmission image, the presence or absence of a seed in the given inspection target 8. Furthermore, if the shape determination unit 86 determines that "a seed is present in the inspection target 8," then the sorting unit 70 may sort that inspection target 8 as a defective article. Thereby, the given inspection target 8 can be classified as seeded or seedless. Namely, it is possible to easily select "seedless" products, which have added value.

In addition, instead of the rejecter parts 60, an air suction type rejecter part may be adopted, wherein the relevant inspection target 8 is suctioned and moved into a target lane.

In addition, guides may be provided at both ends of the plates 21 in the transport direction to provide assistance such that when the given inspection target 8 is pushed out by the relevant rejecter part 60 to the target lane, it is not transported mistakenly to a lane that is not the target.

In addition, the shape determination unit 86 is provided in each of the abovementioned embodiments, but it does not necessarily have to be provided. For example, it does not have to be provided if there is no need to classify according to shape.

GENERAL INTERPRETATION OF TERMS

In understanding the scope of the present invention, the term "comprising" and its derivatives, as used herein, are intended to be open ended terms that specify the presence of the stated features, elements, components, groups, integers, and/or steps, but do not exclude the presence of other unstated features, elements, components, groups, integers and/or steps. The foregoing also applies to words having similar meanings such as the terms, "including", "having" and their derivatives. Also, the terms "part," "section," "portion," "member" or "element" when used in the singular can have the dual meaning of a single part or a plurality of parts. Also as used herein to describe the above embodiment (s), the following directional terms "forward", "rearward", "above", "downward", "vertical", "horizontal", "below" and "transverse" as well as any other similar directional terms refer to those directions of an X-ray inspection apparatus. Accordingly, these terms, as utilized to describe the present invention should be interpreted relative to a device equipped with the X-ray inspection apparatus.

The term "detect" as used herein to describe an operation or function carried out by a component, a section, a device or the like includes a component, a section, a device or the like that does not require physical detection, but rather includes determining, measuring, modeling, predicting or computing or the like to carry out the operation or function. The term "configured" as used herein to describe a component, section or part of a device includes hardware and/or software that is constructed and/or programmed to carry out the desired function. The terms of degree such as "substantially", "about" and "approximately" as used herein mean a reasonable amount of deviation of the modified term such that the end result is not significantly changed.

While only selected embodiments have been chosen to illustrate the present invention, it will be apparent to those skilled in the art from this disclosure that various changes and modifications can be made herein without departing from the scope of the invention as defined in the appended claims. For example, the size, shape, location or orientation of the various components can be changed as needed and/or desired. Components that are shown directly connected or contacting each other can have intermediate structures disposed between them. The functions of one element can be performed by two, and vice versa. The structures and functions of one embodiment can be adopted in another embodiment. It is not necessary for all advantages to be present in a particular embodiment at the same time. Every feature which is unique from the prior art, alone or in combination with other features, also should be considered a separate description of further inventions by the applicant, including the structural and/or functional concepts embodied by such feature(s). Thus, the foregoing descriptions of the embodiments according to the present invention are provided for illustration only, and not for the purpose of limiting the invention as defined by the appended claims and their equivalents.

## Claims

1. An X-ray inspection apparatus comprising:

   an X-ray radiating part configured and arranged to radiate X-rays to an inspection target;
   an X-ray detecting part configured and arranged to detect the X-rays radiated from the X-ray radiating part that transmitted through the inspection target;
   a mass estimation unit configured to estimate a mass of the inspection target based on an amount of the X-rays detected by the X-ray detecting part; and
   a mass class determination unit configured to determine which mass class among a plurality of mass classes within a preset range the inspection target belongs to based on the mass of the inspection target estimated by the mass estimation unit.

2. The X-ray inspection apparatus according to claim 1, wherein
   the mass class determination unit is configured to determine that the mass of the inspection target is abnormal when the mass of the inspection target does not belong to any of the mass classes within the preset range.

3. The X-ray inspection apparatus according to claim 2, further comprising
   a sorting unit configured and arranged to sort the inspection target as a defective article when the mass class determination unit determines that the mass of the inspection target is abnormal.

4. The X-ray inspection apparatus according to an one of claims 1 to 3, further comprising
   a sorting unit configured and arranged to sort the inspection target into a corresponding mass class when the mass class determination unit determines that the inspection target belongs to one of the mass classes.

5. The X-ray inspection apparatus according to any one of claims 1 to 4, wherein
   the X-ray radiating part is configured and arranged to radiate the X-rays to the inspection target from the same direction toward two or more locations of the inspection target or from two or more directions toward at least one location of the inspection target,
   the mass estimation unit is configured to estimate a mass of a useful portion of the inspection target based on the amount of the X-rays detected by the X-ray detecting part, and
   the mass class determination unit is configured to determine which mass class among the mass classes within the preset range the inspection target belongs to based on the mass of the useful portion of the inspection target estimated by the mass estimation unit.

6. The X-ray inspection apparatus according to any one of claims 1 to 5, further comprising
   an image generation unit configured to generate an X-ray transmission image based on the amount of the X-rays detected by the X-ray detecting part, and
   a shape determination unit configured to determine whether a shape of the inspection target is normal or abnormal based on the X-ray transmission image.

7. The X-ray inspection apparatus according to claim 6, further comprising
a sorting unit configured and arranged to sort the inspection target as a defective article when the shape determination unit determines that the shape of the inspection target is abnormal.

8. The X-ray inspection apparatus according to claim 6 or 7, wherein
the shape determination unit is configured to determine whether a seed is present or absent in the inspection target based on the X-ray transmission image when the inspection target is a foodstuff selected from the group consisting of fruits and vegetables.

9. The X-ray inspection apparatus according to claim 8, further comprising
a sorting unit configured and arranged to sort the inspection target as a defective article when the shape determination unit determines that a seed is present in the inspection target.

10. The X-ray inspection apparatus according to any one of claims 1 to 9, wherein
the X-ray radiating part is configured and arranged to radiate the X-rays to the inspection target that is a foodstuff selected from the group consisting of shellfishes and peeled boiled eggs.

11. The X-ray inspection apparatus according to any one of claims 1 to 10, further comprising
a foreign matter determination unit configured to determine whether foreign matter is present or absent in the inspection target based on the amount of the X-rays detected by the X-ray detecting part.

12. The X-ray inspection apparatus according to claim 11, further comprising
a sorting unit configured and arranged to sort the inspection target as a defective article when the foreign matter determination unit determines that foreign matter is present in the inspection target.

# F I G. 1

10

4

1

5

2

20

8

6

7

3

# F I G. 2

F I G. 3

F I G. 4

F I G. 5

7 COMPUTER → X-RAY RADIATING PART — 5

← X-RAY DETECTING PART — 6

71 CPU ↔ DISPLAY AND INPUT UNIT — 4

72 MEMORY → SORTING UNIT — 70

# F I G. 6

COMPUTER — 7

FOREIGN MATTER
DETERMINATION UNIT — 80

MASS INSPECTION UNIT — 81

83 — MASS ESTIMATION
UNIT

84 — MASS CLASS
DETERMINATION UNIT

SHAPE INSPECTION UNIT — 82

85 — IMAGE GENERATION
UNIT

86 — SHAPE
DETERMINATION UNIT

# F I G. 7

83

MASS
ESTIMATION UNIT

S1

84

MASS CLASS
DETERMINATION
UNIT

S2

87

DEFECT
DETERMINATION
UNIT

S5

IMAGE
GENERATION
UNIT

S3

SHAPE
DETERMINATION
UNIT

S4

85

86

# F I G. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008320096 A

- JP 2006308467 A